# FASCICULE DE BREVET EUROPEEN

(11) **EP 0 800 803 B1**
(45) Date de publication et mention de la délivrance du brevet: **16.10.2002**
(21) Numéro de dépôt: 97420060.2
(22) Date de dépôt: 10.04.1997
(51) Int. Cl.: A61F 2/42

(54) **Prothèse de cheville**
Knöchelprothese
Ankle prosthesis

(30) Priorité: 11.04.1996 FR 9604759
(43) Date de publication de la demande: 15.10.1997
(73) Titulaire: TORNIER SA, 38330 Saint-Ismier (FR)
(72) Inventeur: Fournol, Stéphane, 75008 Paris (FR)
(74) Mandataire: Myon, Gérard Jean-Pierre

(56) Documents cités:
- DE-U- 8 812 806
- FR-A- 2 684 291
- FR-A- 2 730 157
- US-A- 3 872 519
- US-A- 4 069 518
- US-A- 4 340 978

## Description

La présente invention a pour objet une prothèse destinée à remplacer l'articulation tibio-tarsienne lorsqu'elle est déficiente ou détruite.

L'articulation de la cheville ou tibio-tarsienne réalise la liaison du pied à la jambe et permet notamment la marche. C'est une articulation trochléenne qui encastre l'astragale dans la pince tibio-péronienne à la manière d'un tenon dans une mortaise. L'élément important de cette articulation est la poulie de l'astragale. Celle-ci est un peu plus large en avant qu'en arrière et elle se met en rapport avec la surface concave du pilon tibial. Cette articulation tibio-tarsienne exécute un seul mouvement qui est la flexion-extension du pied selon la direction sagittale, avec une amplitude de l'ordre de 80°.

Cette articulation subit des contraintes extrêmement importantes, puisqu'elle supporte la totalité du poids du corps, augmenté par l'énergie cinétique lorsque le pied prend contact avec le sol avec une certaine vitesse lors de la marche, de la course ou de la réception d'un saut.

On connaît, par example de FR-A-2 684 291, des prothèses de cheville qui sont destinées à remplacer l'articulation tibio-tarsienne et qui se composent de :
- un plateau tibial ancré dans l'extrémité du tibia et terminé vers l'extérieur par une surface sphérique concave ;
- un plateau astragalien fixé dans l'astragale et comportant selon l'axe sagittal du pied un profil externe composé d'une zone centrale plane allongée comportant un ergot perpendiculaire à ce plan et de deux bords latéraux également plans, mais formant un dièdre obtus avec le plan de la zone centrale ;
- et une pièce intermédiaire dont la face inférieure comporte un profil complémentaire du profil externe du plateau astragalien, ladite face comportant au moins une rainure de forme allongée destinée à recevoir l'ergot dudit plateau, tandis que la face supérieure présente un profil sphérique convexe de même rayon que la surface sphérique concave du plateau tibial de manière à s'emboîter l'une dans l'autre.

Ce genre de prothèse de cheville à trois éléments comporte certains inconvénients en ce qui concerne la limitation axiale de l'élément intermédiaire sur le plateau astragalien, empêchant un bon fonctionnement et une parfaite reproduction de l'articulation.

En outre, la surface articulaire prévue entre le plateau tibial et l'élément intermédiaire n'est pas suffisante dans la définition de son profil pour permettre une parfaite amplitude de la prothèse de cheville.

C'est à ces inconvénients qu'entend plus particulièrement remédier la présente invention.

La présente invention a précisément pour objet une prothèse simple et d'implantation facile par voie chirurgicale qui permet de restaurer l'usage de l'articulation de la cheville lorsque celle-ci est détériorée pour une cause quelconque.

La prothèse de cheville suivant l'invention comprend trois éléments qui coopérent intimement les uns avec les autres pour reproduire parfaitement l'articulation d'une cheville saine.

La prothèse de cheville suivant l'invention est conforme à la revendication 1 ci-annexée.

Selon un aspect avantageux de l'invention, l'élément intermédiaire comporte des bords inclinés pour présenter un profil trapézoïdal.

Un autre avantage de l'invention consiste en ce que l'élément intermédiaire peut comporter, à l'opposé du profil en portion de cylindre, un logement dans lequel coopère un doigt solidaire du plateau astragalien, de manière à limiter les déplacements dans toutes les directions de l'élément intermédiaire.

Enfin, selon un autre aspect avantageux de l'invention, le logement est prévu d'une dimension supérieure à celle du doigt solidaire du plateau astragalien pour permettre un glissement limité de l'élément intermédiaire sur le plateau astragalien.

Le dessin annexé, donné à titre d'exemple, permettra de mieux comprendre l'invention, les caractéristiques qu'elle présente et les avantages qu'elle est susceptible de procurer:
- Figure 1 est une vue en perspective éclatée illustrant les différents éléments composant la prothèse de cheville suivant l'invention.
- Figure 2 est une coupe montrant la prothèse de cheville dans un plan sagittal.
- Figure 3 est une coupe illustrant la prothèse de cheville dans un plan frontal.

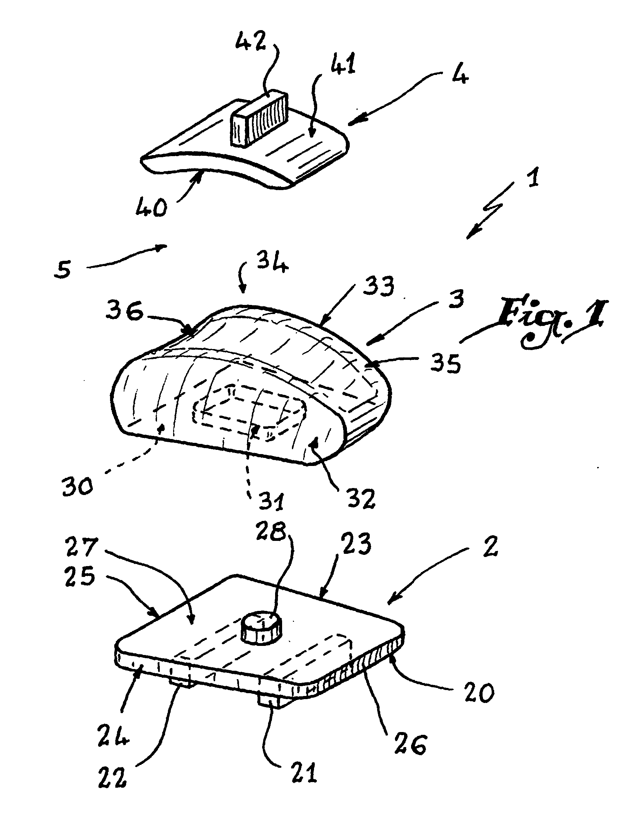

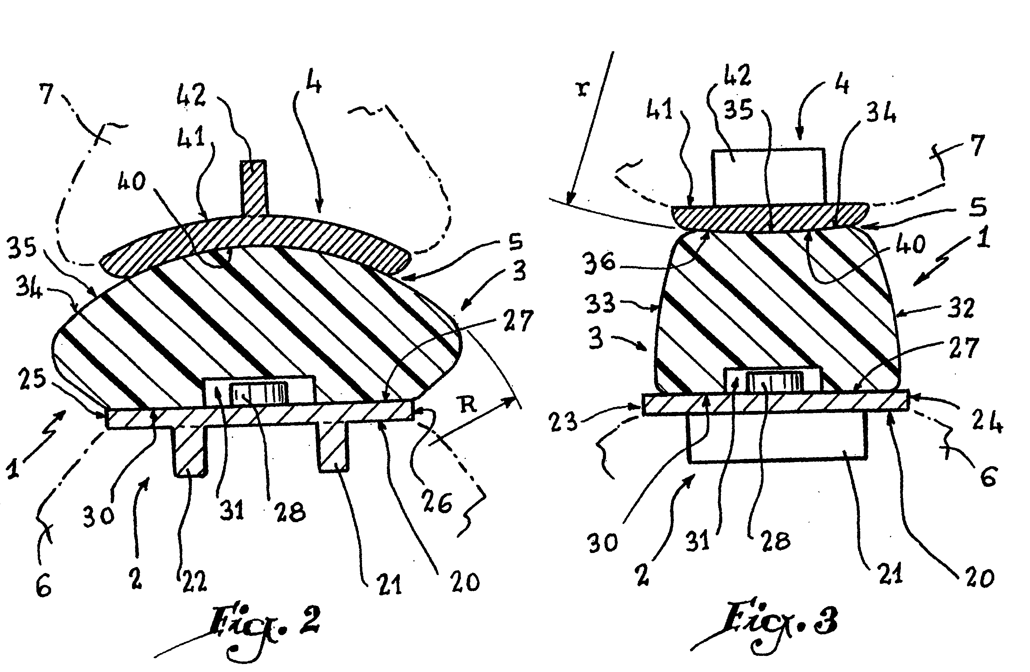

On a représenté en figure 1 à 3 une prothèse de cheville 1 destinée à remplacer l'articulation tibio-tarsienne lorsque celle-ci est détériorée pour une cause quelconque. La prothèse de cheville 1 comporte trois éléments distincts coopérant intimement entre eux pour réaliser l'articulation tibio-tarsienne.

La prothèse de cheville 1 se compose d'un plateau astragalien 2 sur lequel glisse un élément intermédiaire 3 qui forme avec un plateau tibial 4 une surface articulaire 5 reproduisant parfaitement l'articulation d'une cheville.

Le plateau astragalien 2 présente une face inférieure 20 solidaire de deux ergots parallèles 21, 22 permettant sa fixation dans l'astragale 6. Les ergots 21 et 22 sont disposés perpendiculairement à l'axe longitudinal du plateau 2. On note que ce dernier présente un profil trapézoïdal, c'est-à-dire que ses bords 23, 24 de grande longueur ne sont pas parallèles afin de déterminer un bord 25 de plus petite longueur, mais d'une dimension supérieure à celle du bord opposé 26.

Le plateau 2 comprend une face supérieure 27 qui est plane et pourvue en son milieu d'un ergot cylindrique 28 s'étendant verticalement.

L'élément intermédiaire 3 comporte une face inférieure 30 à profil plan, munie en son milieu d'un logement 31.

La face 30 prend appui intimement sur celle 27 du plateau 2, de manière que l'ergot cylindrique 28 pénètre à l'intérieur du logement 31. On remarque que les dimensions du logement 31 sont supérieures à celles de l'ergot 28 de manière que l'élément intermédiaire 3 puisse glisser dans toutes les directions sur le plateau 2 dans un espace délimité.

Bien évidemment, l'espace de glissement de l'élément intermédiaire 3 correspond à la différence des dimensions du logement 31 prévu sur la face inférieure 30 et de l'ergot 28.

L'élément intermédiaire 3 comporte deux bords 32, 33 non parallèles afin qu'il présente un profil trapézoïdal de même ordre que celui prévu pour le plateau astragalien 2.

A l'opposé de la face inférieure 30, l'élément intermédiaire 3 présente une surface supérieure 34 constituant le premier profil de la surface articulaire 5. La surface supérieure 34 comporte un profil en portion de cylindre 35 dont la génératrice 36 est concave dans le plan frontal (figure 3). On note par exemple que le rayon R du cylindre 35 est différent de celui r de la génératrice concave 36. La génératrice 36 est un arc de cercle de rayon r, ce qui permet des débattements du plateau 4 et de l'élément intermédiaire 3 dans le plan frontal. On pourrait prévoir que les rayons R et r soient de dimensions identiques.

On note, par exemple, que le centre du rayon R formant la portion de cylindre 35 est situé dans l'astragale 6 de l'articulation de la cheville. Par contre, le centre du rayon r formant la génératrice concave 36 est situé dans le tibia 7 de l'articulation de la cheville.

Le plateau tibial 4 présente une surface inférieure 40 constituant l'autre profil de la surface articulaire 5. La surface inférieure 40 est conformée suivant un profil complémentaire de celui de la surface supérieure 34 de l'élément intermédiaire 3. Ainsi, la surface 40 vient coopérer avec le profil 35, 36 de l'élément intermédiaire 3 pour réaliser l'articulation tibio-tarsienne. Egalement, le plateau tibial 4 affecte un profil extérieur trapézoïdal du même ordre que ceux de l'élément intermédiaire 3 et du plateau astragalien 2. La face 41 opposée à celle inférieure 40 comporte un ergot 42 s'étendant verticalement à l'intérieur du tibia 7 pour assurer un ancrage parfait du plateau tibial 4. L'ergot 42 est disposé perpendiculairement à l'axe longitudinal du plateau tibial 4 et dans une même direction que ceux 21, 22 prévus sur le plateau astragalien 2.

On constate que le profil trapézoïdal évasé de la pièce intermédiaire 3 implique un sens de montage pour la reproduction de l'articulation anatomique. Ce sens de montage oblige le chirurgien à parfaitement positionner l'élément intermédiaire 3 sur le plateau astragalien 2 pour que l'articulation soit parfaitement reproduite.

## Revendications

1. Prothèse de cheville comprenant un plateau tibial coopérant avec un élément intermédiaire afin de former la surface articulaire de ladite prothèse, tandis que l'élément intermédiaire coopère avec un plateau astragalien apte à être fixé dans l'astragale, **caractérisée en ce qu'**elle comprend une surface articulaire (5) qui est constituée :
- sur l'élément intermédiaire (3), par une surface (34) en forme de cercle (35) en coupe dans le plan sagittal, avec un rayon (R) dont le centre est situé dans l'astragale, et dont la génératrice (36) est un arc de cercle concave dans le plan frontal, avec un rayon (r) dont le centre est placé dans le tibia, et
- sur le plateau tibial (4), par une surface (40) conformée suivant une géométrie complémentaire de celle de ladite surface (34) dudit élément intermédiaire pour réaliser une parfaite coopération.

2. Prothèse de cheville suivant la revendication 1, **caractérisée en ce que** l'élément intermédiaire (3) comporte des bords (32, 33) non parallèles pour présenter un profil trapézoïdal.

3. Prothèse de cheville suivant la revendication 1, **caractérisée en ce que** l'élément intermédiaire (3) comporte, à l'opposé de ladite surface (34) de l'élément intermédiaire, un logement (31) dans lequel coopère un doigt (28) solidaire du plateau astragalien (2) de manière à limiter les déplacements de l'élément intermédiaire (3).

4. Prothèse de cheville suivant la revendication 3, **caractérisée en ce que** le logement (31) est prévu de dimensions plus grandes que celles du doigt (28) pour permettre un glissement limité de l'élément intermédiaire (3) sur le plateau astragalien (2).

5. Prothèse de cheville suivant la revendication 1, **caractérisée en ce que** le rayon (R) de ladite surface (34) de l'élément intermédiaire (3) est identique au rayon (r) délimitant la génératrice concave (36).

6. Prothèse de cheville suivant la revendication 1, **caractérisée en ce que** le rayon (R) de ladite surface (34) de l'élément intermédiaire (3) est différent du rayon (r) délimitant la génératrice concave (36).

7. Prothèse de cheville suivant la revendication 1, **caractérisée en ce que** le plateau astragalien (2) comporte une surface inférieure (20) solidaire d'ergots (21, 22) disposés perpendiculairement à l'axe longitudinal dudit plateau (2).

8. Prothèse de cheville suivant la revendication 7, **caractérisée en ce que** le plateau astragalien (2) présente un profil trapézoïdal, c'est-à-dire que deux de ses bords (23, 24) ne sont pas parallèles.

9. Prothèse de cheville suivant la revendication 1, **caractérisée en ce que** le plateau tibial (4) présente un profil trapézoïdal identique à celui du plateau astragalien (2).

10. Prothèse de cheville suivant la revendication 9, **caractérisée en ce que** le plateau tibial (4) présente sur sa surface supérieure (41) un ergot (42) s'étendant verticalement à l'intérieur du tibia (7) pour permettre sa fixation.

11. Prothèse de cheville suivant la revendication 10, caractérisée en que l'ergot (42) est disposé perpendiculairement à l'axe longitudinal du plateau tibial (4).

## Patentansprüche

1. Knöchelprothese mit einer Schienbeinplatte, die mit einem Zwischenelement zusammenwirkt, um die Gelenkfläche der Prothese zu bilden, während das Zwischenelement mit einer Sprungbeinplatte zusammenwirkt, die dazu ausgelegt ist, im Sprungbein befestigt zu werden, **dadurch gekennzeichnet, dass** die Prothese eine Gelenkfläche (5) umfasst, die folgendermaßen aufgebaut ist:
- am Zwischenelement (3) durch eine Fläche (34) in der Form eines Kreises (35) im Schnitt in der Sagittalebene, mit einem Radius (R), dessen Zentrum sich im Sprungbein befindet, und dessen Mantellinie (36) ein konkaver Kreisbogen in der Stirnebene ist, mit einem Radius (r), dessen Zentrum im Schienbein angeordnet ist, und
- an der Schienbeinplatte (4) durch eine Fläche (40), die gemäß einer Geometrie angeglichen ist, die zu derjenigen der Fläche (34) des Zwischenelements komplementär ist, um ein perfektes Zusammenwirken zu bewerkstelligen.

2. Knöchelprothese nach Anspruch 1, **dadurch gekennzeichnet, dass** das Zwischenelement (3) nicht parallele Ränder (32, 33) umfasst, um ein trapezförmiges Profil auszubilden.

3. Knöchelprothese nach Anspruch 1, **dadurch gekennzeichnet, dass** das Zwischenelement (3), der Fläche (34) des Zwischenelements entgegengesetzt, eine Aufnahme (31) umfasst, in der ein mit der Sprungbeinplatte (2) fest verbundener Zapfen (28) zusammenwirkt, um die Verschiebungen des Zwischenelements (3) zu begrenzen.

4. Knöchelprothese nach Anspruch 3, **dadurch gekennzeichnet, dass** die Aufnahme (31) von den Abmessungen her größer ausgelegt ist als der Zapfen (28), um ein begrenztes Gleiten des Zwischenelements (3) an der Sprungbeinplatte (2) zuzulassen.

5. Knöchelprothese nach Anspruch 1, **dadurch gekennzeichnet, dass** der Radius (R) der Fläche (34) des Zwischenelements (3) identisch zum Radius (r) ist, der die konkave Mantellinie (36) abgrenzt.

6. Knöchelprothese nach Anspruch 1, **dadurch gekennzeichnet, dass** sich der Radius (R) der Fläche (34) des Zwischenelements (3) vom Radius (r), der die konkave Mantellinie (36) abgrenzt, unterscheidet.

7. Knöchelprothese nach Anspruch 1, **dadurch gekennzeichnet, dass** die Sprungbeinplatte (2) eine untere Fläche (20) umfasst, die fest mit Stiften (21, 22) verbunden ist, die senkrecht zur Längsachse der Platte (2) angeordnet sind.

8. Knöchelprothese nach Anspruch 7, **dadurch gekennzeichnet, dass** die Sprungbeinplatte (2) ein trapezförmiges Profil aufweist, d.h. dass zwei ihrer Ränder (23, 24) nicht parallel sind.

9. Knöchelprothese nach Anspruch 1, **dadurch gekennzeichnet, dass** die Schienbeinplatte (4) ein trapezförmiges Profil aufweist, das zu demjenigen der Sprungbeinplatte (2) identisch ist.

10. Knöchelprothese nach Anspruch 9, **dadurch gekennzeichnet, dass** die Schienbeinplatte (4) an ihrer oberen Fläche (41) einen Stift (42) aufweist, der sich vertikal ins Innere des Schienbeins (7) erstreckt, um seine Befestigung zuzulassen.

11. Knöchelprothese nach Anspruch 10, **dadurch gekennzeichnet, dass** der Stift (42) senkrecht zur Längsachse der Schienbeinplatte (4) angeordnet ist.

## Claims

1. Ankle prosthesis comprising a tibial platform cooperating with an intermediate element to form the articular surface of the said prosthesis, while the intermediate element cooperates with an astragalian platform for fixing into the astragalus, **characterized in that** it comprises an articular surface (5) which is constituted:
- on the intermediate element (3), by a surface (34) in the shape of a circle (35) in section in the sagittal plane, with a radius (R) whose centre is situated in the astragalus, and whose generatrix (36) is an arc of a circle concave in the frontal plane, with a radius (r) whose centre is located in the tibia, and
- on the tibial platform (4), by a surface (40) shaped with a geometry complementing that of the said surface (34) of the said intermediate element so as to provide perfect cooperation.

2. Ankle prosthesis according to Claim 1, **characterized in that** the intermediate element (3) has non-parallel sides (32, 33) so that it possesses a trapezoidal profile.

3. Ankle prosthesis according to Claim 1, **characterized in that** the intermediate element (3) has, opposing the said surface (34) of the intermediate element, a recess (31) in which a peg (28) integral with the astragalian platform (2) cooperates so as to limit the movements of the intermediate element (3).

4. Ankle prosthesis according to Claim 3, **characterized in that** the recess (31) is given larger dimensions than those of the peg (28) to allow limited sliding of the intermediate element (3) on the astragalian platform (2).

5. Ankle prosthesis according to Claim 1, **characterized in that** the radius (R) of the said surface (34) of the intermediate element (3) is the same as the radius (r) defining the concave generatrix (36).

6. Ankle prosthesis according to Claim 1, **characterized in that** the radius (R) of the said surface (34) of the intermediate element (3) is different from the radius (r) defining the concave generatrix (36).

7. Ankle prosthesis according to Claim 1, **characterized in that** the astragalian platform (2) has a lower surface (20) integral with lugs (21, 22) disposed perpendicularly to the longitudinal axis of the said platform (2).

8. Ankle prosthesis according to Claim 7, **characterized in that** the astragalian platform (2) possesses a trapezoidal profile, i.e. **in that** two of its sides (23, 24) are not parallel.

9. Ankle prosthesis according to Claim 1, **characterized in that** the tibial platform (4) possesses the same trapezoidal profile as the astragalian platform (2).

10. Ankle prosthesis according to Claim 9, **characterized in that** the tibial platform (4) possesses on its upper surface (41) a lug (42) extending vertically inside the tibia (7), for fixing.

11. Ankle prosthesis according to Claim 10, **characterized in that** the lug (42) is disposed perpendicularly to the longitudinal axis of the tibial platform (4).
